# DEMANDE DE BREVET EUROPEEN

(11) **EP 2 093 226 A1**
(43) Date de publication de la demande: **26.08.2009**
(21) Numéro de dépôt: 08172189.6
(22) Date de dépôt: 18.12.2008
(51) Int. Cl.: C07D 471/04, A61Q 5/10

(54) **Nouvelles aminoindolizines, composition tinctoriale comprenant une aminoindolizine, procedes et utilisations**

(30) Priorité: 20.12.2007 FR 0760136
(71) Demandeur: L'Oreal, 75008 Paris (FR)
(72) Inventeur: Vidal, Laurent, 75013, PARIS (FR); Metais, Eric, 95320, Saint Leu La Foret (FR); Fadli, Aziz, 77500, Chelles (FR); Katritzki, Alan, 32601, Gainsville (FR)
(74) Mandataire: Eveillard, Sophie

(57) **Abrégé**

La présente demande concerne une nouvelle famille d'aminoindolizines, de formule (I) : 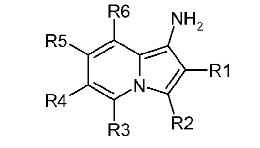 leur utilisation pour la teinture des fibres kératiniques, en particulier des fibres kératiniques humaines telles que les cheveux, les compositions tinctoriales comprenant de telles aminoindolizines ainsi que les utilisations de ces aminoindolizines.

## Description

La présente demande a pour objet de nouvelles aminoindolizines, leur utilisation pour la teinture des fibres kératiniques, en particulier des fibres kératiniques humaines telles que les cheveux, les compositions tinctoriales comprenant de telles aminoindolizines ainsi que les procédés mettant en oeuvre ces aminoindolizines.

Il est connu de teindre les fibres kératiniques et en particulier les cheveux humains avec des compositions tinctoriales contenant des précurseurs de colorants d'oxydation, appelés généralement bases d'oxydation, tels que des ortho- ou para-phénylènediamines, des ortho- ou para-aminophénols et des composés hétérocycliques. Ces bases d'oxydation sont des composés incolores ou faiblement colorés qui, associés à des produits oxydants, peuvent donner naissance à des composés colorés par un processus de condensation oxydative.

On sait également que l'on peut faire varier les nuances obtenues avec ces bases d'oxydation en les associant à des coupleurs ou modificateurs de coloration, ces derniers étant choisis notamment parmi les méta-diaminobenzènes aromatiques, les méta-aminophénols, les méta-diphénols et certains composés hétérocycliques tels que des composés indoliques.

La variété des molécules mises en jeu au niveau des bases d'oxydation et des coupleurs permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (c'est-à-dire abîmée) entre sa pointe et sa racine.

De manière surprenante et avantageuse, la Demanderesse vient de découvrir qu'il est possible d'obtenir de nouvelles compositions pour la teinture des fibres kératiniques, en particulier des fibres kératiniques humaines telles que les cheveux, capables de conduire à des colorations aux nuances variées, puissantes, esthétiques, peu sélectives et résistant bien aux diverses agressions que peuvent subir les fibres, en utilisant au moins une aminoindolizine.

En outre, ces compositions présentent un bon profil toxicologique.

Un premier objet de l'invention concerne une famille d'aminoindolizines et leurs procédés de synthèse.

L'invention a aussi pour objet une composition contenant au moins une aminoindolizine, les procédés de teinture mettant en oeuvre cette composition, les utilisations de ladite composition selon la présente invention pour la teinture des fibres kératiniques, en particulier les fibres kératiniques humaines telles que les cheveux et en particulier, les dispositifs à plusieurs compartiments ou "kits" de teinture.

La composition de la présente invention permet en particulier d'obtenir une coloration des fibres kératiniques très puissante, peu sélective et tenace, en particulier à la lumière.

D'autres caractéristiques, aspects, objets et avantages de la présente invention apparaîtront encore plus clairement à la lecture de la description et des exemples qui suivent.

La présente invention concerne une aminoindolizine de formule générale (I), ses sels d'addition avec un acide et ses solvates : 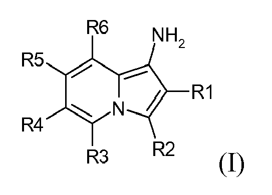 dans laquelle
- R₁ est un radical choisi parmi :
   - les radicaux alkyles en C₁-C₁₀, linéaires ou ramifiés, éventuellement substitués par un ou plusieurs groupements OH ou OR₁₉, R₁₉ étant choisi parmi les radicaux alkyles en C₁-C₆, linéaires ou ramifiés, éventuellement substitués par un ou plusieurs radicaux OH,
   - les radicaux soufrés choisis parmi SR, SO₂Me, R étant un radical alkyle en C₁-C₆, linéaire ou ramifié, ou un radical aryle,
   - les radicaux azotés choisis parmi NH₂, NHR₇ ou NR₇R₈, R₇ et R₈ identiques ou différents, étant choisis parmi les radicaux alkyles en C₁-C₆, linéaires ou ramifiés, éventuellement substitués par un ou plusieurs groupes hydroxy, amino, mono- ou dialkylamino en C₁-C₆, carboxamido, ureido ou guanidinyle, éventuellement interrompus par un atome d'oxygène ou un ou plusieurs groupes N(R₁₃), R₁₃ étant un atome d'hydrogène ou un radical alkyle en C₁-C₆, les radicaux R₇ et R₈ pouvant former ensemble avec l'atome d'azote auquel ils sont rattachés un hétérocycle insaturé ou saturé comprenant de 5 à 7 chaînons,
   - le radical OR₉, R₉ étant choisi parmi les radicaux alkyles en C₁-C₆, linéaires ou ramifiés, éventuellement substitués par un ou plusieurs radicaux OH, ou les radicaux alcoxy en C₁-C₆, linéaires ou ramifiés,
   - les radicaux choisis parmi COOH, CONH₂, CONHR₁₁, ou CONR₁₁R₁₂, R₁₁ et R₁₂ ,identiques ou différents, étant choisis parmi les radicaux alkyles en C₁-C₆, linéaires ou ramifiés, éventuellement interrompus par un ou plusieurs groupements N(R₁₄), R₁₄ étant un atome d'hydrogène ou un radical alkyle en C₁-C₆, éventuellement substitué par un groupement hydroxy, les radicaux R₁₁ et R₁₂ pouvant former ensemble et avec l'atome d'azote auquel ils sont rattachés un hétérocycle insaturé ou saturé comprenant de 5 à 7 chaînons ;
- R₂ est un radical choisi parmi :
   - les radicaux alkyles en C₁-C₆, linéaires ou ramifiés,
   - l'atome d'hydrogène,
   - les radicaux choisis parmi COOH, CN, CF₃ ou alcoxycarbonyle COOR₁₀, R₁₀ étant choisi parmi les radicaux alkyles en C₁-C₆, linéaires ou ramifiés, les radicaux aryle ou hétéroaryle,
   - les radicaux choisis parmi CONH₂, CONHR₁₁, ou CONR₁₁R₁₂,
   - le radical OR₉.
   - les radicaux SR, SOR, ou SO₂R,
   R, R₉, R₁₁ et R₁₂ étant tels que définis précédemment,
- R₃, R₄, R₅, R₆, indépendamment l'un de l'autre, sont choisis parmi:
   - les radicaux aryles, hétéroaryles, ou alkyles en C₁-C₆, linéaires ou ramifiés, éventuellement interrompus par un ou plusieurs groupements N(R₁₃) ou par un oxygène,
   - le radical OR₉,
   - l'atome d'hydrogène,
   - les halogènes choisis parmi le fluor, le chlore, ou le brome,
   - les radicaux azotés choisis parmi NH₂, NHR₇ ou NR₇R₈,
   - les radicaux COOH, CN ou alcoxy carbonyle COOR₁₀,
   R₇, R₈, R₉ et R₁₀ étant tels que définis précédemment,
à la condition qu'au plus un seul des radicaux R₁, R₃, R₄, R₅, R₆ représente un radical azoté choisis parmi NH₂, NHR₇ ou NR₇R₈.

De préférence, R₁ est choisi parmi :
- les radicaux alkyles en C₁-C₁₀, linéaires ou ramifiés, de préférence C₁-C₆ et plus préférentiellement C₁-C₄,
- les radicaux soufrés SO₂Me,
- les radicaux azotés choisis parmi NHR₇ ou NR₇R₈, R₇ et R₈ identiques ou différents, étant choisis parmi les radicaux alkyles en C₁-C₆, linéaires ou ramifiés, éventuellement substitués par un ou plusieurs groupes hydroxy, amino, mono ou dialkylamino en C₁-C₆, de préférence hydroxy, mono ou dialkylamino en C₁-C₄.

De manière préférée, R₁ est choisi parmi les radicaux suivants : - CH₃, -SO₂CH₃, -NH(CH₃), -N(CH₂CH₂OH)₂, -N(CH₃)₂, - NH(CH₂CH₂OH), -NH(CH₂CH₂N(CH₃)₂), -NH(CH₂CH₃), - NH(CH₂CH₂CH₂N(CH₃)₂).

De préférence, le radical R₂ est choisi parmi :
- les radicaux alkyles en C₁-C₆, linéaires ou ramifiés, de préférence en C₁-C₄,
- l'atome d'hydrogène,
- les radicaux choisis parmi COOH, CN, CF₃ ou alcoxycarbonyle COOR₁₀, R₁₀ étant choisi parmi les radicaux alkyles en C₁-C₆, linéaires ou ramifiés,
- les radicaux choisis parmi CONH₂, CONHR₁₁, ou CONR₁₁R₁₂,
- le radical OR₉,
- les radicaux SR ou SO₂R.

De manière préférée, le radical R₂ est choisi parmi l'atome d'hydrogène ou les radicaux suivants: -CH₃, -COOCH₃, -COOCH₂CH₃, - CN, -CF₃, - CONH(CH₂CH₃), -CONH(CH₂CH₂CH₂N(CH₃)₂), - CON(CH₃)₂, -CONH(CH₂CH₂OH), -CONH₂, -OCH₃, -SCH₃, -SO₂CH₃.

De préférence, les radicaux R₃, R₄, R₅, R₆, indépendamment l'un de l'autre, sont choisis parmi:
- l'atome d'hydrogène,
- le radical OR₉ avec R₉ étant un radical alkyle C₁-C₄, de préférence - OCH₃,
- le radical -NH₂, à la condition qu'au plus un seul des radicaux R₁, R₃, R₄, R₅, R₆ représente un radical -NH₂.

Les aminoindolizines de formule (I) peuvent se présenter sous forme libre ou sous forme de sels, tel que les sels d'addition avec un acide minéral ou organique, de préférence choisis parmi les chlorhydrates, les bromhydrates, les sulfates, les citrates, les succinates, les tartrates, les lactates, les tosylates, les benzènesulfonates, les phosphates et les acétates.

De préférence, les aminoindolizines de formule (I) sont choisies parmi les composés suivants :

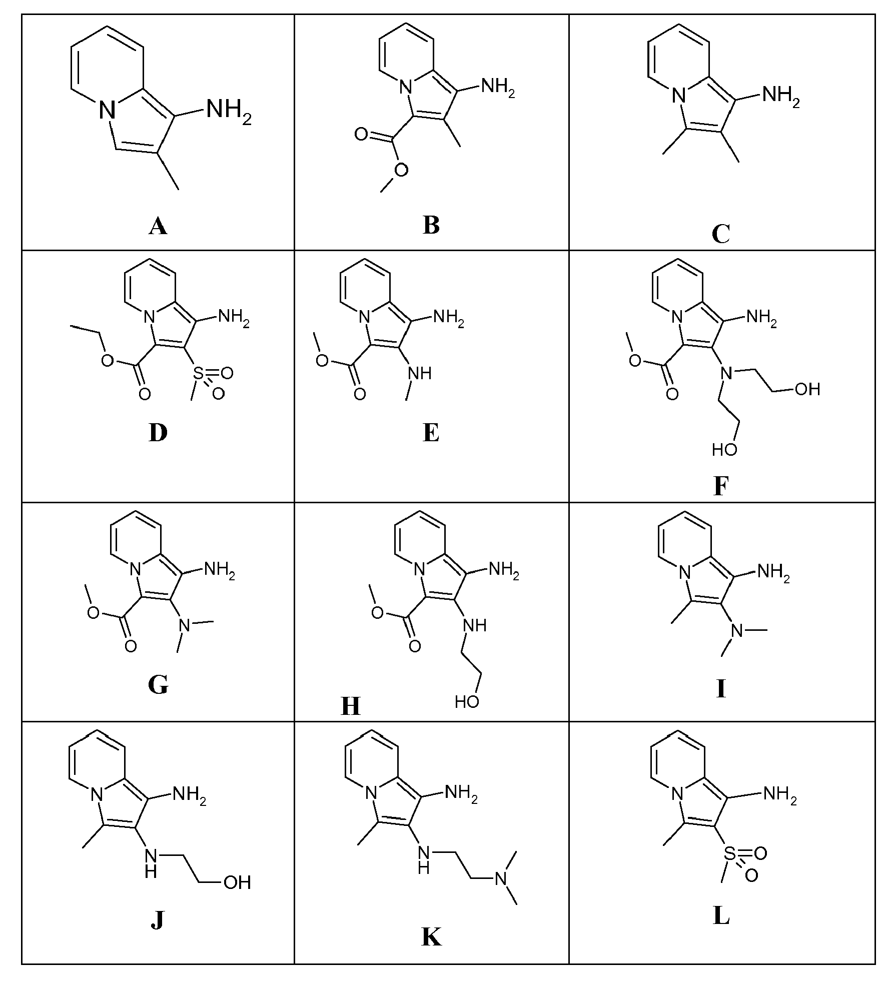

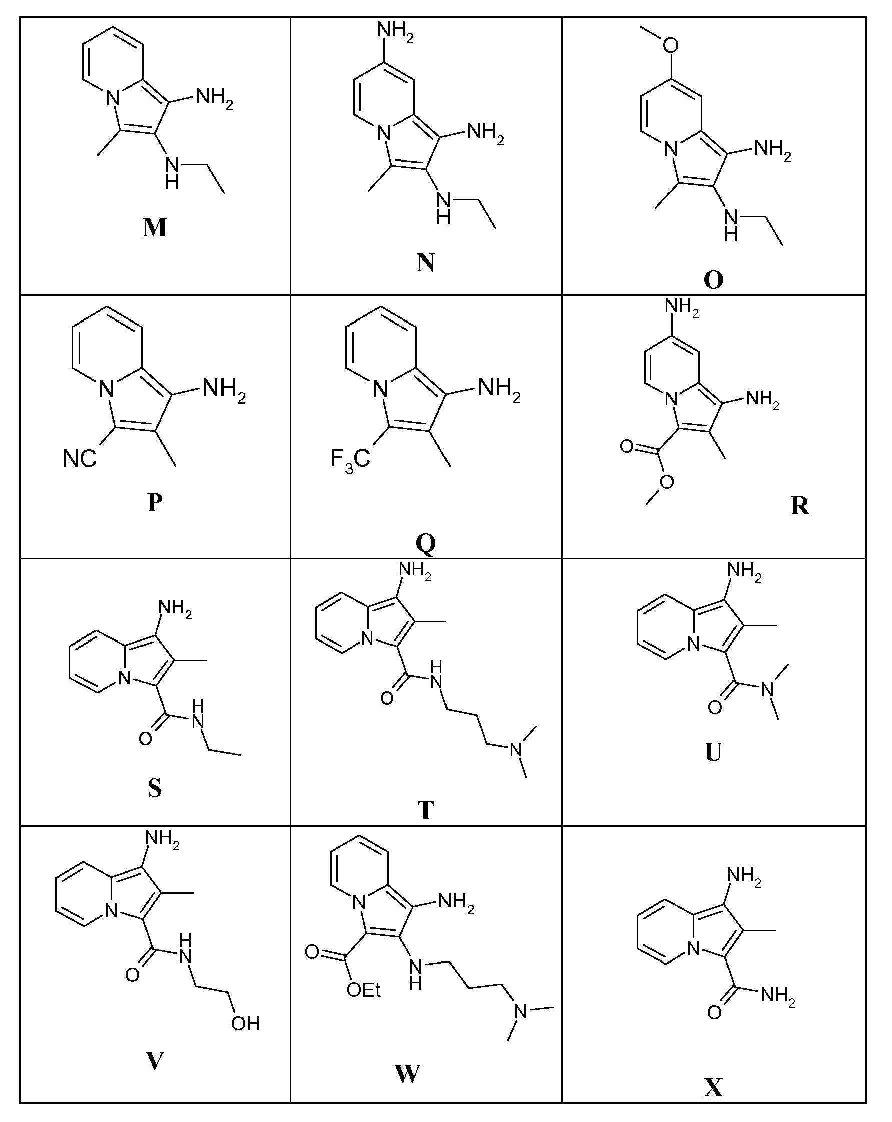

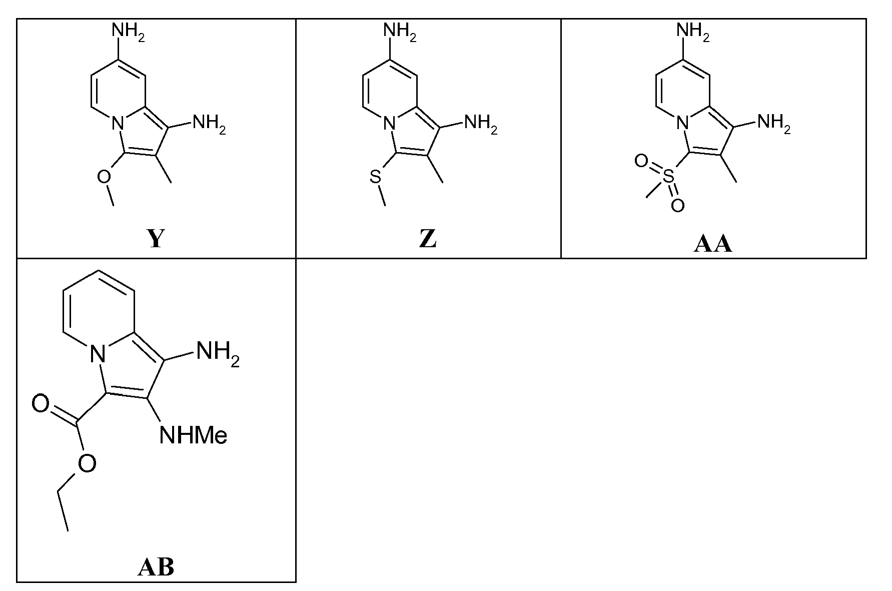

ainsi que leurs sels d'addition et/ou solvates.

Les aminoindolizines de formule (I) selon la présente demande peuvent être préparées suivant différentes voies de synthèse.

La présente demande concerne encore un procédé de synthèse d'une aminoindolizine de formule générale (I) dans lequel on effectue une réduction ou une déacylation de l'aminoindolizine de formule générale (II) correspondante : 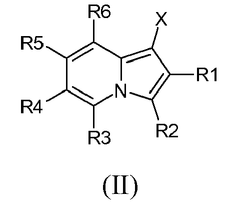 dans laquelle :
- X est choisi parmi
   - les radicaux nitro, nitroso, arylazo, hétéroarylazo,
   - les radicaux NHCOR₁₅, R₁₅ étant un radical alkyle en C₁-C₆, linéaire ou ramifié, aryle ou hétéroaryle,
- R₁, R₂, R₃, R₄, R₅, R₆ sont tels que définis précédemment.

### Voie 1 : Condensation d'une alpha-halogénocétone ou alpha-alcoxycétone avec un dérivé d'alpha-picoline

### Première variante :

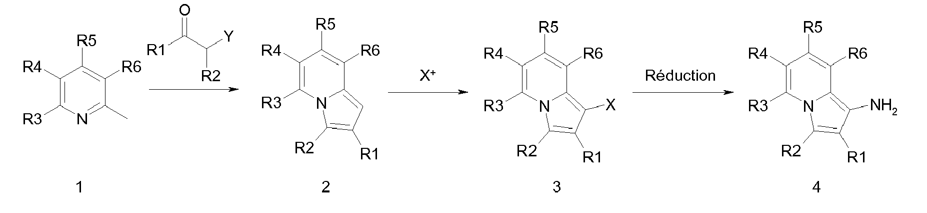

La première étape consiste à condenser une cétone, portant un halogène (Y) en alpha, sur un dérivé d'alpha-picoline 1 (Batroff V. ; Flitsch W., Liebigs Annalen der Chemie, 1987, (7), p.621). L'indolizine 2 obtenue est susceptible de réagir avec tout électrophile X⁺ précurseur d'amine par réduction du type NO⁺, NO₂⁺, ArN₂⁺ ou HetArN₂⁺ pour conduire aux composés (3). Ceux-ci sont ensuite soumis à une réaction d'hydrogénation par catalyse hétérogène telle que Pd/C, Pd(II)/C, Ni/Ra, etc... ou encore à une réaction de réduction par un métal tel que par du zinc, fer, étain,etc...( voir Advanced Organic Chemistry, 3ème édition, J. March, et Réduction in organic Chemistry, M ; Hudlicky) pour conduire aux indolizines (4).
Cette voie est valable lorsque :
- X représente :
   - un radical nitro, nitroso ou arylazo ou hétéroarylazo et
- R₁ représente :
   - un radical alkyle en C₁-C₁₀, linéaire ou ramifié, éventuellement substitués par un ou plusieurs groupements OH ou OR₁₉ ou
   - un radical de type COOH, CONH₂, CONHR₁₁, ou CONR₁₁R₁₂, et
- R₂ représente :
   - un radical alkyle en C₁-C₆, linéaire ou ramifié.
   - un atome d'hydrogène,
   - un radical de type CN ou CF₃ et
- R₃, R₄, R₅, R₆, indépendamment l'un de l'autre, représentent :
   - un radical aryle, hétéroaryle, ou alkyle en C₁-C₆, linéaire ou ramifié, éventuellement interrompu par un ou plusieurs groupements N(R₁₃) ou par un atome d'oxygène,
   - un atome d'hydrogène,
   - un radical OR₉,
   - un halogène choisi parmi le fluor, le chlore, ou le brome,
   - un radical azoté choisi parmi NH₂, NHR₇ ou NR₇R₈,
   - un radical de type COOH, CN ou alcoxy carbonyle COOR₁₀.
   R₇,R₈, R₉,R₁₀, étant tels que définis précédemment.
Les exemples de synthèse 4 et 5 sont conformes à cette variante.

### Deuxième variante

- Lorsque R₂ représente un radical de type COOH, ou alcoxycarbonyle COOR₁₀, R₁, R₃, R₄, R₅, R₆ étant tels que définis ci-dessus :
   il est nécessaire d'ajouter une étape de carboxylation après l'étape d'obtention des indolizines 2b (Bobrovskii, S. I. et coll., Mosk. Gos. Univ., Moscow, USSR. Khimiya Geterotsiklicheskikh Soedinenii 1989, (12), 1634-8) :

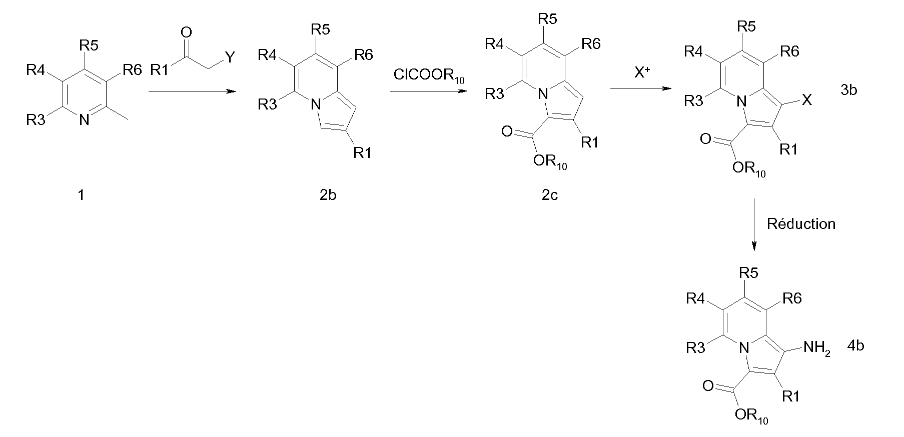

L'exemple de synthèse 1 est conforme à cette variante.

Le radical de type COOH est obtenu à partir du composé 3b par saponification de la fonction ester (Eur. J. of. Org. Chem., 2001, p. 3705).

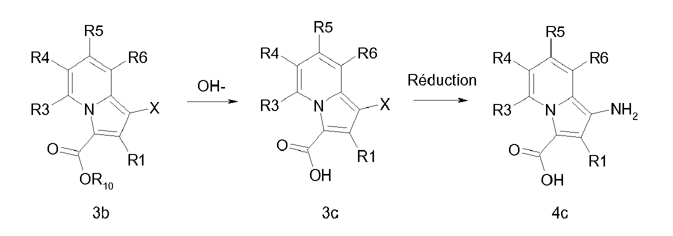

### Troisième variante

- Lorsque R₂ représente un radical de type CN , R₁, R₃, R₄, R₅, R₆ étant tels que définis ci-dessus :
   Il est possible d'obtenir le composé directement à partir du composé 2b (Smaliy, R. V. et coll., Synthesis, 2002, (16), p.2416).

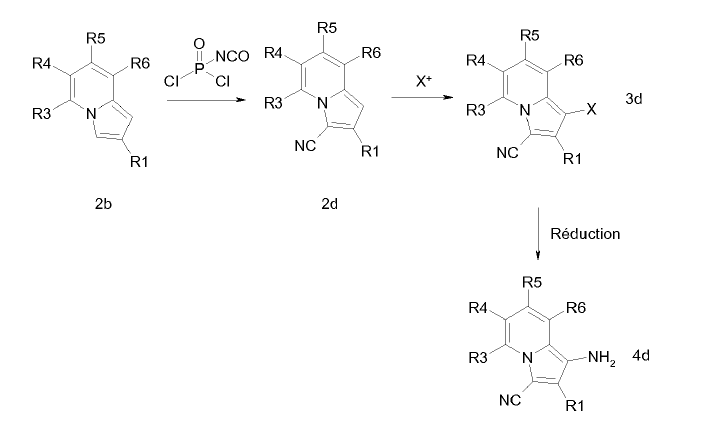

### Quatrième variante

- Lorsque R₂ représente un radical de type CONH₂, CONHR₁₁, ou CONR₁₁R₁₂ , R₁, R₃, R₄, R₅, R₆ étant tels que définis ci-dessus :
   il est nécessaire de convertir la fonction ester des indolizines 3b en fonction amide soit par amination directe (revue : Beckwith, in Zabicky, The Chemistry of Amides ; Wiley : New-York, 1970, p. 86-96) ou soit par saponification de la fonction ester en acide carboxylique puis par traitement par diverses amines (revue : Beckwith, in Zabicky, The Chemistry of Amides, p. 105-109).

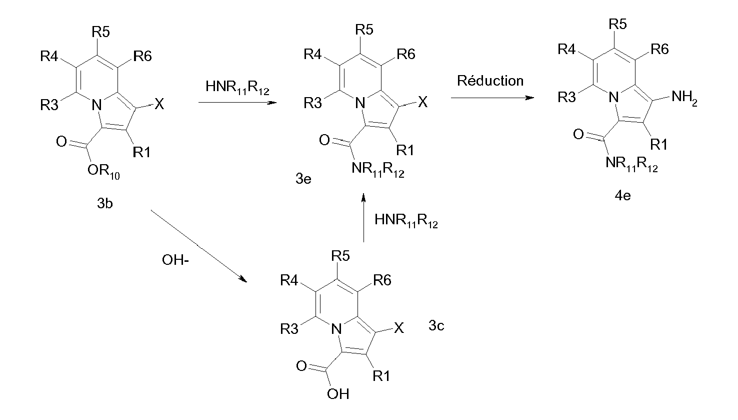

### Cinquième variante

- Lorsque R₂ représente un radical de type SR, SOR, SO₂R , R₁, R₃, R₄, R₅, R₆ étant tels que définis ci-dessus :
   il existe plusieurs méthodes pour introduire un groupement SR en position 3 des indolizines du type 2b :
      - Soit par réaction avec un disulfure (Eur. Pat. Appl. 350384, 1990 et Synthesis, 1980, (11), p. 886-7) :

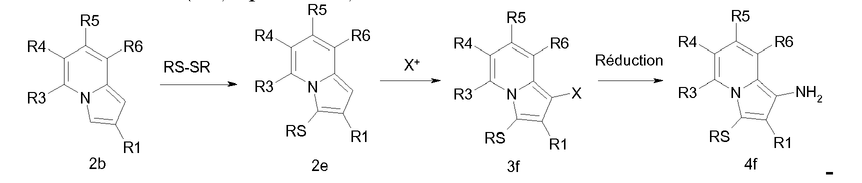

Soit par réaction avec un dérivé soufré (Synthesis, 1980, (11), p. 886-7) :

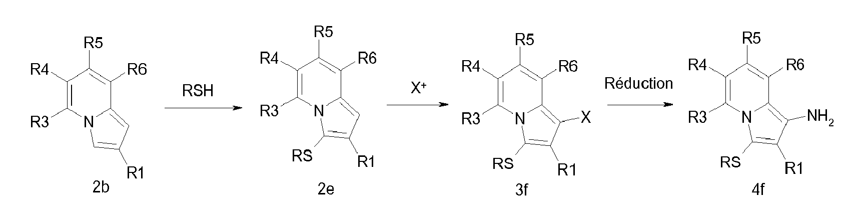

La conversion d'un groupement SR en SOR ou SO₂R en position 3 est réalisable à partir des indolizines du type 3fpar oxydation à l'aide par exemple d'acides perbenzoïques (Eur. Pat. Appl. 350384, 1990) :

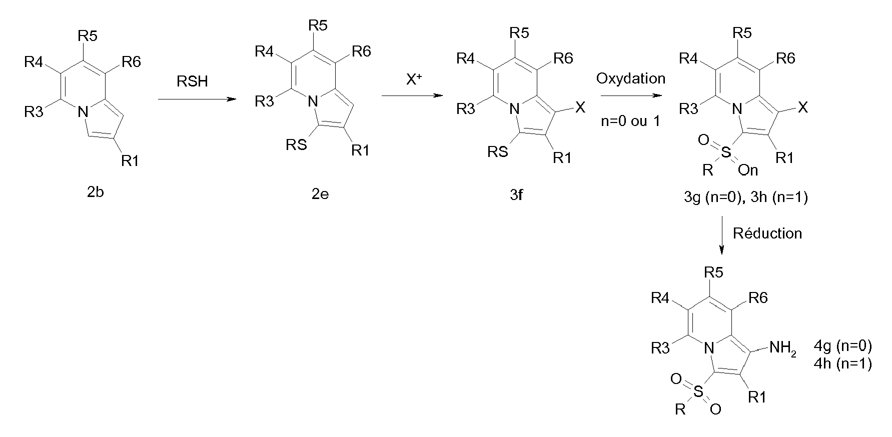

### Sixième variante

- Lorsque R₂ représente un radical de type OR₉ , R₁, R₃, R₄, R₅, R₆ étant tels que définis ci-dessus :
   il est possible d'utiliser le mode opératoire suivant :

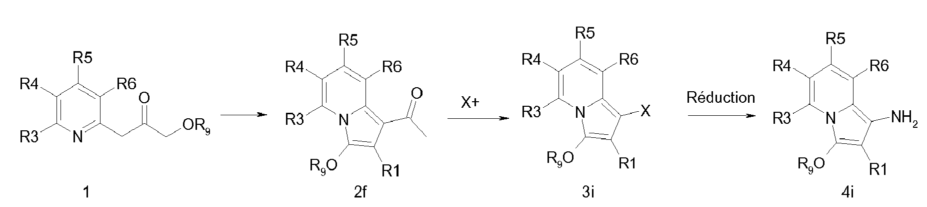

Les 2 premières étapes sont décrites par Léonard, N. J. et coll. (J. Org. Chem., 1957, 22, p.1445) et Colonna, M. et coll. (J. Chem. Soc., PT2 : Phys. Org. Chem., 1984, (2), p. 165), lorsque X+=NO₂.

Pour qu'un seul des groupes R₃, R₄, R₅, ou R₆, représente un radical NH₂ : Il est important de prévoir le précurseur de l'amine dès le départ,
- soit en partant d'une aminopicoline, en prenant soin de protéger l'amine, 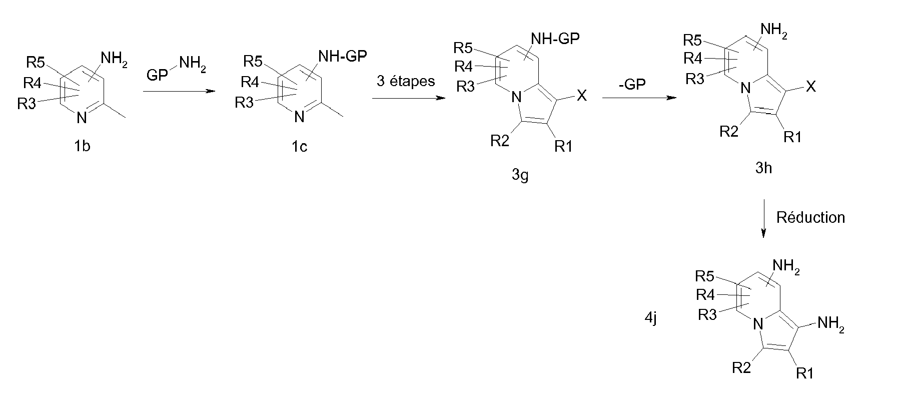 L'étape de déprotection intervient avant l'étape de réduction finale. Dans le cas où le groupe protecteur est un dérivé benzylique, la réduction, par hydrogénation catalytique sur Pd-C, peut servir d'étape de déprotection.
- soit en introduisant un atome d'halogène qui peut conduire ensuite par substitution ou par couplage organométallique. 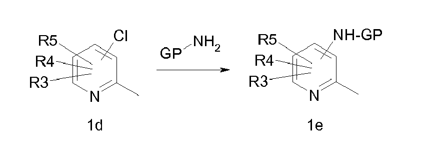
- soit en partant d'une nitropicoline, l'amine étant obtenue lors de l'étape de réduction.

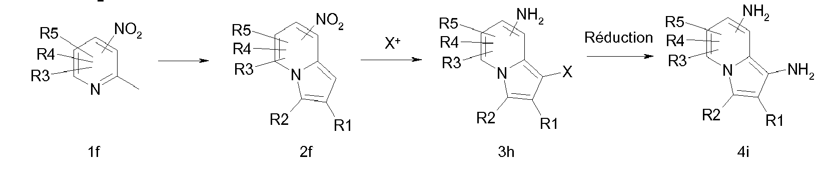

**Voie 2 :**Condensation d'un dérivé alpha-halogéno acétate d'alkyle ou alpha-halogénoacétonitrile avec une pyridine puis cyclisation par réaction avec un dérivé de méthylthio-2-nitroéthène

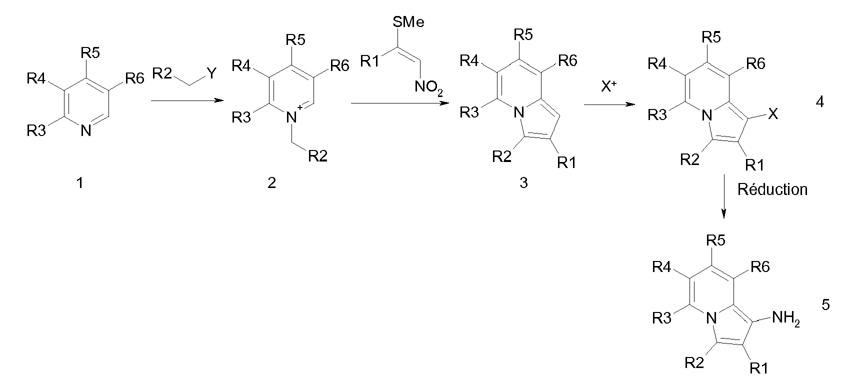

Cette voie est valable lorsque :
- X représente un radical nitro, nitroso ou azo (arylazo ou hétéroarylazo),
- R₁ représente :
   - un radical alkyle en C₁-C₁₀, linéaire ou ramifié,
   - un radical soufré choisi parmi SR, R étant un radical alkyle en C₁-C₆, linéaire ou ramifié, ou aryle.
   - un radical azoté choisi parmi NHR₇ ou NR₇R₈.
- R₂ représente :
   - un radical de type COOH, CN ou alcoxycarbonyle COOR₁₀.
- R₃, R₄, R₅, R₆, représentent :
   - un radical aryle, hétéroaryle, ou alkyle en C₁-C₆, linéaire ou ramifié, éventuellement interrompu par un ou plusieurs groupements N(R₁₃) ou par un oxygène,
   - un atome d'hydrogène,
   - un radical OR₉,
   - un halogène choisi parmi le fluor, le chlore, ou le brome.
   - un radical azoté choisi parmi NH₂ sous une forme protégée, NHR₇ ou NR₇R₈.
   - un radical de type COOH, CN ou alcoxycarbonyle COOR₁₀,
R₇,R₈, R₉,R₁₀, étant tels que définis précédemment.

Dans ce cas, la diversité du groupe R₁ peut être apportée selon 2 voies :
- Soit à partir du bis-méthylthio-2-nitroéthène : 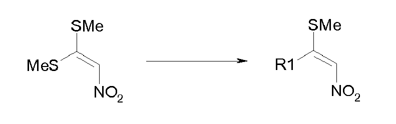 qui permet ainsi d'accéder aux R₁ suivants :
   - un radical alkyle en C₁-C₁₀, linéaire ou ramifié, selon Teran, N. et coll. Tetrahedron, 1998, 54(42), p. 12973-84.
   - un radical soufré choisi parmi SR, selon Rao, H. et coll., Tet. Lett., 2003, 44(25), p.4701-4.
   - un radical azoté choisi parmi NHR₇ ou NR₇R₈, selon par exemple Wermuth, C.G. et coll., J. Med. Chem., 1999, 42(4), 730-41 ; Ootsuka Y. et coll., Jpn. Kokai Tokkyo Koho, 07157465, 1995 et Manjunatha, S. G. et coll., Tet. Lett., 1990, 31(9), 1327-30.
- Soit après oxydation du dérivé soufré 3a obtenu par cyclisation du pyridinium 2 avec le bis-méthylthio-2-nitroéthène selon : 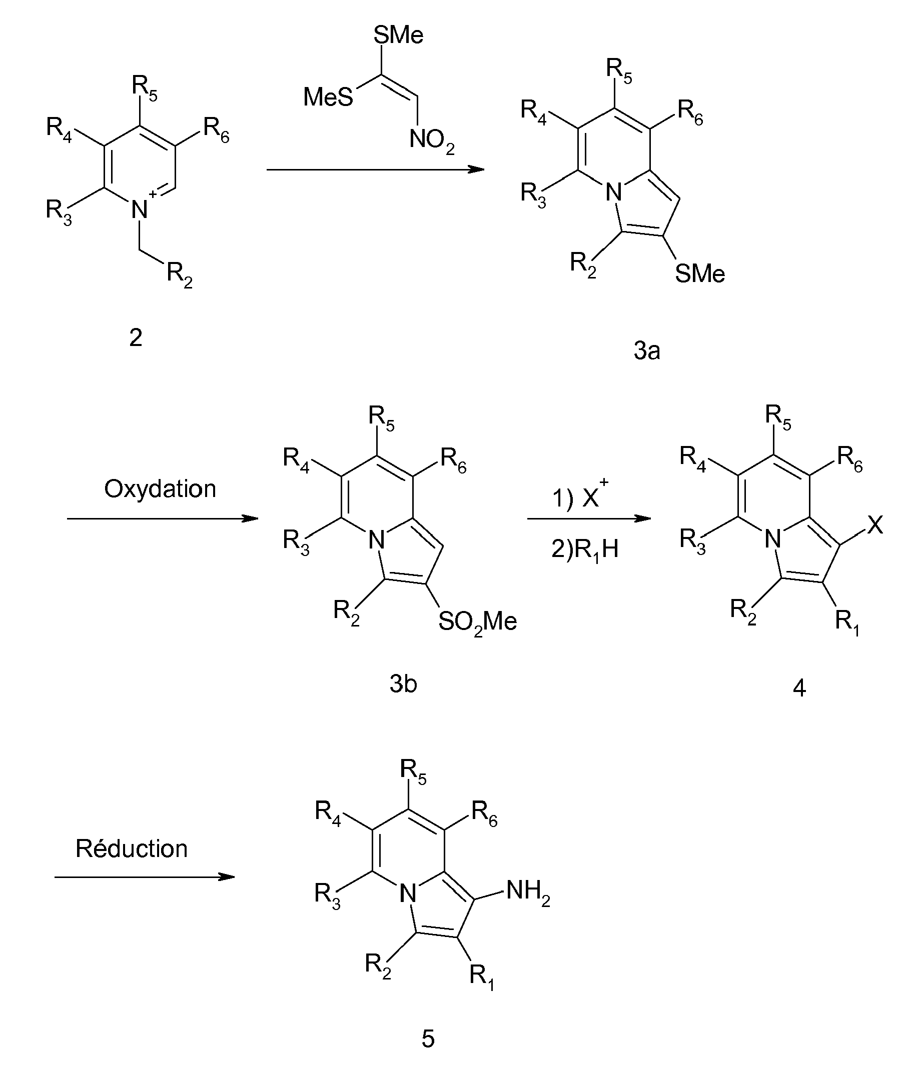 qui permet par substitution du groupe sulfonyle intermédiaire 3b d'accéder aux R₁ suivants :
   - un radical azoté choisi parmi NH₂, NHR₇ ou NR₇R₈.
   - un radical OR₉.

• Pour qu'un seul des groupes R₃, R₄, R₅, ou R₆, représente un radical NH₂ , R₁ devra être impérativement différent de H.

Il est important de prévoir le précurseur de l'amine dès le départ,
- soit en partant d'une aminopyridine, en prenant soin de protéger l'amine à l'aide d'un groupe benzylique (Andrews, A.F., J. Chem. Soc., PT1 : Org. And Bio. Chem (1982), (12), 2995-3006 et Isin, E. M, JOC, (2001), 66(12), 4220-6) 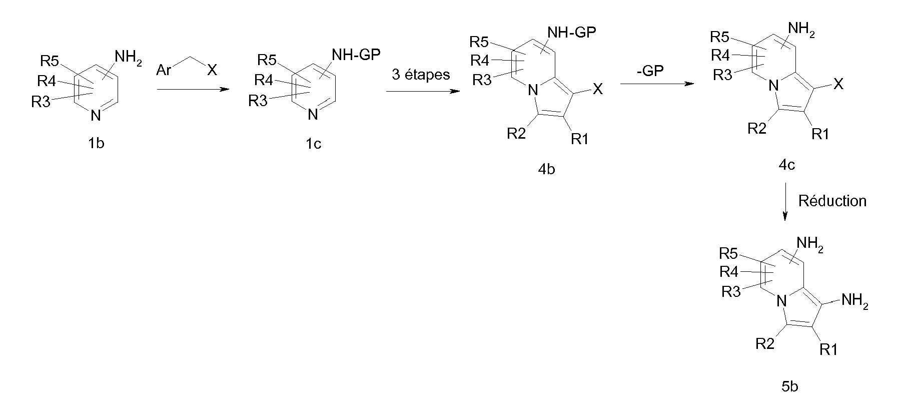 L'étape de déprotection devra intervenir avant l'étape de réduction finale. Dans le cas où le groupe protecteur est un dérivé benzylique, la réduction, par hydrogénation catalytique sur Pd-C, peut servir d'étape de déprotection.
- soit en introduisant un atome d'halogène qui peut conduire ensuite par substitution (Shimizu, K. et coll., Jpn Kokai Tokkyo Koho (2001) JP 2001151753) ou par couplage organométallique (Desmarets, C. et coll, JOC, (2002), 67(9), 3029-36). 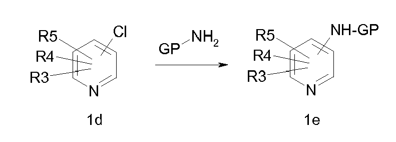
- soit en partant d'une nitropyridine, l'amine étant obtenue lors de l'étape de réduction.

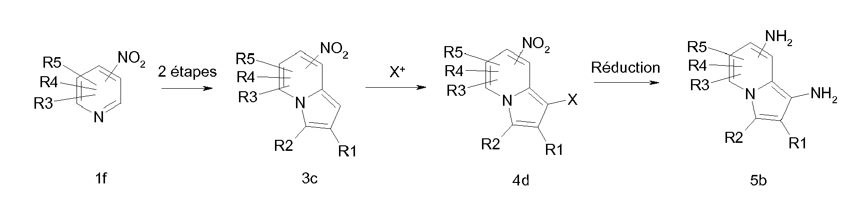

La présente demande concerne également les aminoindolizines de de formule générale (II) : 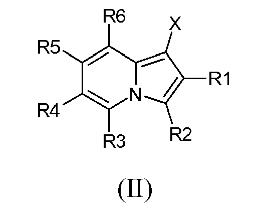 dans laquelle :
- X est choisi parmi
   - les radicaux nitro, nitroso, arylazo, hétéroarylazo,
   - les radicaux NHCOR₁₅, R₁₅ étant un radical alkyle en C₁-C₆, linéaire ou ramifié, aryle ou hétéroaryle,
- R₁ est un radical choisi parmi :
   - les radicaux alkyles en C₁-C₁₀, linéaires ou ramifiés, éventuellement substitués par un ou plusieurs groupements OH ou OR₁₉, R₁₉ étant choisi parmi les radicaux alkyles en C₁-C₆, linéaires ou ramifiés, éventuellement substitués par un ou plusieurs radicaux OH,
   - les radicaux soufrés choisis parmi SR, SO₂Me, R étant un radical alkyle en C₁-C₆, linéaire ou ramifié, ou un radical aryle,
   - les radicaux azotés choisis parmi NH₂, NHR₇ ou NR₇R₈, R₇ et R₈ identiques ou différents, étant choisis parmi les radicaux alkyles en C₁-C₆, linéaires ou ramifiés, éventuellement substitués par un ou plusieurs groupes hydroxy, amino, mono- ou dialkylamino en C₁-C₆, carboxamido, ureido ou guanidinyle, éventuellement interrompus par un atome d'oxygène ou un ou plusieurs groupes N(R₁₃), R₁₃ étant un atome d'hydrogène ou un radical alkyle en C₁-C₆, les radicaux R₇ et R₈ pouvant former ensemble avec l'atome d'azote auquel ils sont rattachés un hétérocycle insaturé ou saturé comprenant de 5 à 7 chaînons,
   - le radical OR₉, R₉ étant choisi parmi les radicaux alkyles en C₁-C₆, linéaires ou ramifiés, éventuellement substitués par un ou plusieurs radicaux OH, ou les radicaux alcoxy en C₁-C₆, linéaires ou ramifiés,
   - les radicaux choisis parmi COOH, CONH₂, CONHR₁₁, ou CONR₁₁R₁₂, R₁₁ et R₁₂, identiques ou différents, étant choisis parmi les radicaux alkyles en C₁-C₆, linéaires ou ramifiés, éventuellement interrompus par un ou plusieurs groupements N(R₁₄), R₁₄ étant un atome d'hydrogène ou un radical alkyle en C₁-C₆, éventuellement substitués par un groupement hydroxy les radicaux R₁₁ et R₁₂ pouvant former ensemble et avec l'atome d'azote auquel ils sont rattachés un hétérocycle insaturé ou saturé comprenant de 5 à 7 chaînons,
- R₂ est un radical choisi parmi :
   - les radicaux alkyles en C₁-C₆, linéaires ou ramifiés,
   - l'atome d'hydrogène,
   - les radicaux choisis parmi COOH, CN, CF₃ ou alcoxycarbonyle COOR₁₀, R₁₀ étant choisi parmi les radicaux alkyles en C₁-C₆, linéaires ou ramifiés, les radicaux aryle ou hétéroaryle,
   - les radicaux choisis parmi CONH₂, CONHR₁₁, ou CONR₁₁R₁₂,
   - le radical OR₉.
   - les radicaux SR, SOR, ou SO₂R,
   R, R₉, R₁₁ et R₁₂ étant tels que définis précédemment,
- R₃, R₄, R₅, R₆, indépendamment l'un de l'autre, sont choisis parmi:
   - les radicaux aryles, hétéroaryles, ou alkyles en C₁-C₆, linéaires ou ramifiés, éventuellement interrompus par un ou plusieurs groupements N(R₁₃) ou par un oxygène,
   - le radical OR₉, avec R₉ étant un radical alkyle C₁-C₄, de préférence - OCH₃,
   - l'atome d'hydrogène,
   - les halogènes choisis parmi le fluor, le chlore, ou le brome.
   - les radicaux azotés choisis parmi NH₂, NHR₇ ou NR₇R₈,
   - les radicaux COOH, CN ou alcoxycarbonyle COOR₁₀,
   R₇, R₈, R₉, R₁₀ et R₁₃ étant tels que définis précédemment,
à la condition qu'au plus un seul des radicaux R₁, R₃, R₄, R₅, R₆ représente un radical azoté choisis parmi NH₂, NHR₇ ou NR₇R₈,
à l'exclusion des composés suivants :

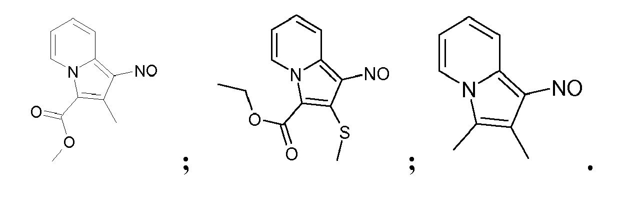

La présente demande concerne aussi les utilisations d'une aminoindolizine de formule générale (I) et/ou ses sels ou solvates pour la teinture des fibres kératiniques, notamment les fibres kératiniques humaines telles que les cheveux.

La présente demande concerne aussi une composition cosmétique de teinture, notamment des fibres kératiniques telles que les cheveux, comprenant, dans un milieu approprié pour la teinture, au moins une aminoindolizine de formule générale (I) et/ou ses sels ou solvates.

De préférence, la concentration de l'aminoindolizine de formule générale (I) est comprise entre 0,0001 et 20%, de préférence entre 0,005 à 6% en poids par rapport au poids total de la composition.

Le milieu approprié pour la teinture comprend généralement de l'eau ou un mélange d'eau et d'au moins un solvant organique tel que, par exemple, les alcools inférieurs en C₁-C₄, ramifiés ou non, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, le glycérol ainsi que les alcools aromatiques comme l'alcool benzylique ou le phénoxyéthanol, et leurs mélanges.

Avantageusement, la composition cosmétique comprend au moins un adjuvant cosmétique choisi dans le groupe formé par les agents antioxydants, les agents de pénétration, les agents séquestrants, les parfums, les tampons, les agents dispersants, les tensioactifs, les conditionneurs, les agents filmogènes, les polymères, les céramides, les agents conservateurs, les agents nacrants ou opacifiants, les vitamines ou provitamines.

Les adjuvants ci-dessus sont en général présents en quantité comprise pour chacun d'eux entre 0,01 et 20 % en poids par rapport au poids de la composition.

La composition selon l'invention contient de préférence au moins un coupleur d'oxydation.

Parmi ces coupleurs d'oxydation, on peut notamment citer les méta-phénylènediamines, les métaaminophénols, les méta-diphénols, les coupleurs naphtaléniques et les coupleurs hétérocycliques ainsi que leurs sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène (ou résorcinol), le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6-hydroxy benzomorpholine, la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

Généralement la concentration du ou des coupleurs d'oxydation est comprise entre 0,0001 et 20%, de préférence entre 0,005 à 6% en poids par rapport au poids total de la composition.

La composition peut aussi contenir au moins une base d'oxydation additionnelle différente des aminoindolizines de formule (I). Ces bases peuvent notamment être choisies parmi les para-phénylènediamines, les bis-phénylalkylènediamines, les para-aminophénols, les ortho-aminophénols et les bases hétérocycliques et leurs sels d'addition.

Parmi les para-phénylènediamines, on peut plus particulièrement citer à titre d'exemple, la para-phénylènediamine, la para-toluènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthyloxy para-phénylènediamine, la 2-β-acétylaminoéthyloxy para-phénylènediamine, la N-(β-méthoxyéthyl) para-phénylènediamine, la 4 aminophenyl pyrrolidine, le 2 thiényl para-phénylène diamine, le 2-β hydroxyéthylamino 5-amino toluène, la 3-hydroxy 1-(4' aminophenyl)pyrrolidine, la 6-(4-Amino-phenylamino)-hexan-1-ol et leurs sels d'addition avec un acide.

Parmi les para-phénylènediamines citées ci-dessus, la para-phénylènediamine, la para-toluènediamine, la 2-isopropyl para-phénylènediamine, la 2-β-hydroxyéthyl para-phénylènediamine, la 2-β-hydroxyéthyloxy para-phénylènediamine, la 2,6-diméthyl para-phénylènediamine, la 2,6-diéthyl para-phénylènediamine, la 2,3-diméthyl para-phénylènediamine, la N,N-bis-(β-hydroxyéthyl) para-phénylènediamine, la 2-chloro para-phénylènediamine, la 2-β-acétylaminoéthyloxy para-phénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para--aminophénol, le 4-amino 3-méthyl phénol, le 4-amino 3-fluoro phénol, le 4-amino 2-chloro phénol, le 4-amino 3-chloro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, le 4-amino-2,6 dichlorophénol, le 4-amino 6[((5'amino-2'hydroxy-3'-méthyl)phényl)méthyl]-2-méthylphénol, le bis[(5'amino-2'hydroxy)phénylméthane et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ;
ainsi que leurs sels d'addition avec un acide.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition.

Généralement la concentration de la ou des bases d'oxydation additionnelles différentes des aminoindolizines de formule générale (I) est comprise entre 0,0001 et 20%, de préférence entre 0,005 à 6% en poids par rapport au poids total de la composition.

D'une manière générale, les sels d'addition avec un acide utilisables pour les bases d'oxydation et les coupleurs sont notamment choisis parmi les chlorhydrates, les bromhydrates, les sulfates, les citrates, les succinates, les tartrates, les lactates, les tosylates, les benzènesulfonates, les phosphates et les acétates.

La composition tinctoriale conforme à l'invention peut en outre contenir un ou plusieurs colorants directs pouvant notamment être choisis parmi les colorants nitrés de la série benzénique, neutres, acides ou cationiques, les colorants directs azoïques neutres, acides ou cationiques, les colorants directs quinoniques et en particulier anthraquinoniques neutres, acides ou cationiques, les colorants directs aziniques, les colorants directs méthiniques, azométhiniques, triarylméthaniques, indoaminiques et les colorants directs naturels. De préférence, la composition selon l'invention comprend au moins un colorant choisi parmi les colorants directs cationiques et les colorants directs naturels.

Parmi les colorants directs cationiques utilisables selon l'invention on peut citer les colorants directs azoïques cationiques décrits dans les demandes de brevets WO 95/15144, WO-95/01772 et EP-714954.

Parmi ces composés on peut tout particulièrement citer les colorants suivants :
- chlorure de 1,3-diméthyl-2-[[4-(diméthylamino)phényl]azo]-1H-Imidazolium,
- chlorure de 1,3-diméthyl-2-[(4-aminophényl)azo]-1H-Imidazolium,
- méthylsulfate de 1-méthyl-4-[(méthylphénylhydrazono) méthyl]-pyridinium.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Le ou les colorants directs représentent de préférence de 0,001 à 20% en poids environ du poids total de la composition et encore plus préférentiellement de 0,005 à 10% en poids environ.

Bien entendu, l'homme de l'art veillera à choisir le ou les adjuvants, précurseurs de colorants d'oxydations additionnels, colorants directs de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques autres que les diacides carboxyliques comme l'acide chlorhydrique, l'acide ortho-phosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule : 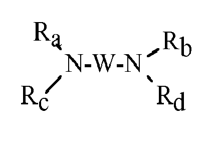 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition cosmétique selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

La présente demande concerne un procédé de teinture des fibres kératiniques dans lequel on applique sur les fibres kératiniques la composition selon la présente invention telle que définie précédemment pendant une durée suffisante pour développer la coloration désirée en présence d'un agent oxydant, l'agent oxydant étant appliqué avant, simultanément ou après la composition.

La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration.

Selon ce mode de réalisation particulier, on dispose d'une composition prête à l'emploi qui est un mélange d'une composition selon l'invention avec au moins un agent oxydant. Le mélange obtenu, est ensuite appliqué sur les fibres kératiniques pendant une durée suffisante pour développer la coloration désirée. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

La présente demande a aussi pour objet un procédé de teinture des fibres kératiniques dans lequel on applique sur lesdites fibres la composition prête à l'emploi pendant une durée suffisante pour développer la coloration désirée.

La durée suffisante pour développer la coloration désirée correspond généralement à un temps de pose de 3 à 50 minutes environ, de préférence 5 à 30 minutes environ.

L'invention a aussi pour objet un dispositif à plusieurs compartiments ou "kit" de teinture dans lequel un premier compartiment renferme la composition tinctoriale définie ci-dessus et un deuxième compartiment renferme une composition oxydante. Ce dispositif peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de teindre les fibres kératiniques à partir d'un procédé qui comprend le mélange d'une composition tinctoriale conforme à l'invention avec un agent oxydant tel que défini précédemment, et l'application du mélange obtenu sur les fibres kératiniques pendant un temps suffisant pour développer la coloration désirée.

Les exemples qui suivent servent à illustrer l'invention sans toutefois présenter un caractère limitatif.

### EXEMPLES

### Exemples de synthèse

### Exemple 1 : synthèse du 2-méthyl-1-aminoindolizine-3-carboxylate de méthyle (4) (composé B):

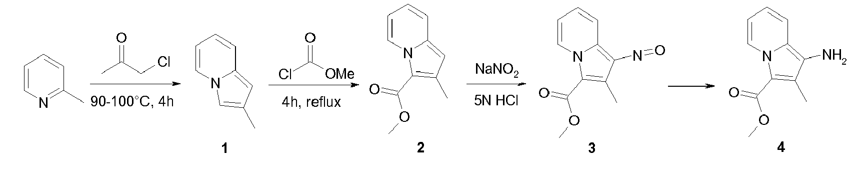

**Préparation de la 2-Méthylindolizine (1).** Un mélange d'α-picoline (5g, 53,8 mmoles) et de chloroacétone (5g, 53,8 mmoles) est chauffé à 90-100°C pendant 4h. Le mélange réactionnel est ensuite versé sur un mélange chloroforme-eau (50:50mL) puis extrait. La phase aqueuse est traitée avec 0,6g de bicarbonate de sodium, puis extraite avec de l'éther diéthylique (3×30mL) pour éliminer l'α-picoline résiduelle. 8,1g (96,4 mmoles) supplémentaires de bicarbonate de sodium sont ajoutés à la phase aqueuse et le mélange est concentré pour obtenir un mélange de précipité blanc et d'eau. L'extraction du mélange avec de l'éther diéthylique conduit à 5,6g de 2-methylindolizine 1 sous forme de cristaux blancs; PF : 57-58 °C. ¹H RMN (CDCl₃): δ 7,77-7,74 (m, 1H), 7,23 (d, J = 8,9 Hz, 1H), 7,06 (s, 1H), 6,58-6,53 (m, 1H), 6,36-6,31 (m, 1H), 6,21 (s, 1H), 2,31 (s, 3H), ¹³C RMN (CDCl₃): δ 132,8, 124,8, 124,6, 118,2, 116,5, 111,1, 109,4, 99,7, 12,4.

**Préparation du 2-méthyl-indolizine-3-carboxylate de méthyle (2).** Un mélange de 2-methylindolizine 1 (3,8 g, 29,0 mmoles) et de chloroformiate de méthyle (22 g, (18ml), 231,6 mmoles) est porté au reflux pendant 2h. 12,2 g (10ml) supplémentaires de chloroformiate de méthyle sont ajoutés et le mélange porté au reflux pendant 2h. Le mélange réactionnel est concentré sous vide puis purifié par chromatographie sur colonne de silice (hexane-acétate d'éthyle 5:1, Gel de silice 200-425 mesh) pour donner 4g de composé 2 (huile incolore). ¹H RMN (CDCl₃): δ 9,43 (d, J = 7,1 Hz, 1H), 7,35 (d, J = 8,8 Hz, 1H), 6,99-6,93 (m, 1H), 6,73-6,68 (m, 1H), 6,29 (s, 1H), 3,91 (s, 3H), 2,53 (s, 3H), ¹³C RMN (CDCl₃): δ 162,7, 137,0, 134,6, 127,6, 121,8, 117,6, 111,8, 103,5, 103,48, 50,6, 15,1.

**Préparation du 2-méthyl-1-nitrosoindolizine-3-carboxylate de méthyle (3).** Une solution de nitrite de sodium (2,2 g, 31,90 mmoles) dans l'eau (10 mL) est ajoutée lentement à 0-5°C, à une solution agitée de 2-méthylindolizine-3-carboxylate de méthyle 2 (3,9 g, 20,61 mmoles), dans l'acide acétique glacial (50 mL). Après 30 min, le milieu réactionnel marron est versé sur l'eau. Des cristaux verts sont collectés par filtration, lavés à l'eau puis séchés sous vide pour conduire à 3,7g de composé attendu; PF : 131-132 °C. ¹H RMN (CDCl₃): 9,63 (d, J=7,0 Hz, 1H), 8,39 (d, J=8,5 Hz, 1H), 7,74-7,69 (m, 1H), 7,23-7,18 (m, 1H), 4,03 (s, 3H), 3,26 (s, 3H), ¹³C NMR (CDCl₃): δ 162,5, 154,5, 141,7, 135,7, 127,9, 123,2, 118,4, 118,3, 114,5, 51,7, 11,1. Anal. Calculée C₁₁H₁₀N₂O₃: C, 60,55; H, 5,54; N, 12,84, trouvé: C, 60,65; H, 5,60; N, 12,90.

**Préparation du 2-méthyl-1-aminoindolizine-3-carboxylate de méthyle (4).** Dans un tricol de 250 mL, équipé d'une agitation magnétique, d'un thermomètre et d'un réfrigérant sont chargés successivement 96 mL d'éthanol, 5mL d'eau et 4 g poudre de zinc. Le milieu réactionnel est porté au reflux et on coule une solution 260mg de chlorure d'ammonium dans 2ml d'eau. On introduit ensuite par petites portions à la spatule 1,09g (5 mmoles) de 2-méthyl-1-nitroso-indolizine-3-carboxylate de méthyle en 30 minutes. Le reflux est maintenu pendant une heure.
Les sels de zinc sont éliminés par filtration sur Celite. Les jus sont acidifiés à l'aide d'une solution d'isopropanol-chlorhydrique puis concentrés sous vide.
Le solide pâteux ainsi obtenu est repris avec un minimum de méthanol et précipité avec de l'éther diisopropylique.

Le solide blanc beige formé est essoré sur fritté n°4 puis séché sous vide en présence de P₂O₅. Après séchage, on récupère une masse de 0,8g de produit attendu.
Les analyses par spectrométrie de masse et spectroscopie RMN sont conformes à la structure attendue:
Les ions quasi-moléculaires (MH)+ , (MNa)+ de la molécule attendue, C₁₁H₁₂N₂O₂, sont principalement détectés.
¹H NMR (DMSO-d6): δ 3.86 (s, 3H), 7. 02(Massif, 1H), 7.28 (m, 1H), 7.92 (m, 1H), 9.37 (m, 1H), 10.3 (se, 3H).

### Exemple 2 : synthèse du 2-méthylamino-1-aminoindolizine-3-carboxylate d'éthyle (8) (composé AB):

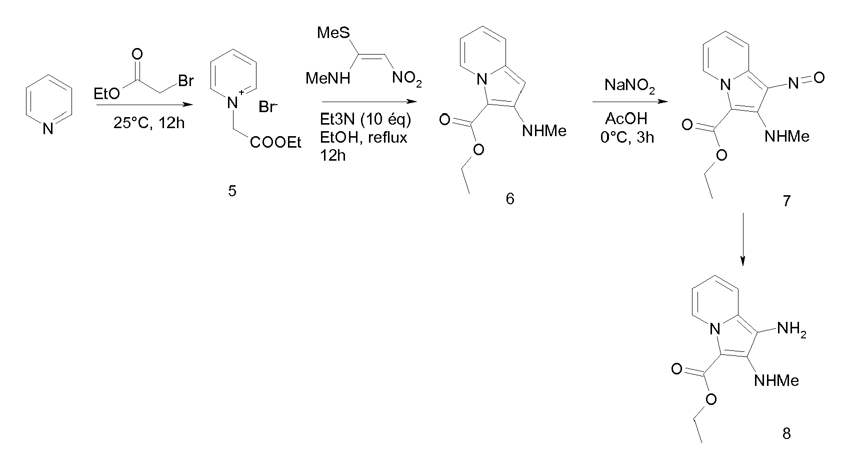

### Préparation du bromure de 1-éthoxycarbonylméthylpyridinium (5) [2003, J. Med. Chem, p.4872]

Un mélange de pyridine (3g, 38 mmoles) et de bromoacétate d'éthyle (6,3g, 38 mmoles) dans 30 ml d'acétate d'éthyle est agité pendant 12h. Le précipité formé est filtré et lavé avec de l'éther diéthylique (30ml) pour conduire après séchage à 7g de bromure de 1-éthoxycarbonylméthylpyridinium 5.

### Préparation du 2-méthylaminoindolizine-3-carboxylate d'éthyle (6).

Une mélange de bromure de 1-éthoxycarbonylméthylpyridinium 5 (1g, 4,1 mmoles), de 1,1-bisméthylthio-2-nitroéthène (1,2g, 8,1 mmoles) et de Et₃N (4,1g, 40,6 mmoles) dans l'éthanol (30mL) est porté au reflux pendant 12h. Les solvants et la triéthylamine sont ensuite évaporés sous vide. L'huile obtenue est extraite avec de l'eau (50mL) et du toluène (3x50mL). Les extraits rassemblés sont séchés avec du sulfate de magnésium anhydre, filtrés puis concentrés sous vide. Le residu est chromatographié sur colonne de silice avec un gradient d'élution 1/1 à 4/1 d'acétate d'éthyle/éther de pétrole. On obtient ainsi 0,5g de 2-méthylaminoindolizine-3-carboxylate d'éthyle 6 sous forme d'huile. ¹H RMN (DMSO-d₆) δ: 9,11 (br s, 1H), 7,32 (d, J = 8,8 Hz, 1H), 7,07-7,01 (m, 1H), 6,71-6,66 (m, 1H), 5,88 (br s, 1H), 5,85 (s, 1H), 4,32 (q, J = 7,0 Hz, 2H), 2,85 (d, J = 5,1 Hz, 3H), 1,34 (t, J = 7,0 Hz, 1H), ¹³C RMN (CDCl₃) δ: 161,0, 148,9, 138,2, 127,0, 123,4, 115,8, 109,9, 98,6, 84,3, 58,7, 31,0, 14,7.

### Préparation de l'ester d'éthyle de l'acide 2-méthylamino-1-nitrosoindolizine-3-carboxylate d'éthyle (7).

Le 2-(méthylamino)-3-indolizinecarboxylate d'éthyle 6 (4,45 g, 20,4 mmoles) est dissous dans CH₃COOH (40 mL) et refroidi à 0 °C avant l'addition d'une solution de nitrite de sodium (1,7 g, 24,0 mmoles) dans l'eau (10 mL). Après agitation à 0 °C pendant 3 h, le mélange réactionnel est basifié avec une solution 2 M de NaOH (pH = 8-9) et extrait avec du CH₂Cl₂ (3x50 mL). Les extraits rassemblés sont séchés avec du sulfate de magnésium anhydre, filtrés puis concentrés sous vide. Le residu est chromatographié sur gel de silice avec un gradient d'élution 1/1 à 4/1 d'acétate d'éthyle/éther de pétrole pour donner après élimination des solvants 3,89 g de microcristaux verts de 2-(méthylamino)-1-nitroso-3-indolizinecarboxylate d'éthyle 7, PF : 156-157 °C. ¹H RMN (DMSO-d₆): δ 1,40 (t, J = 7,0 Hz, 3H), 3,47 (d, J = 5,4 Hz, 3H), 4,38 (q, J = 7,0 Hz, 2H), 7,35 (t, *J* = 7,0 Hz, 1H), 7,23 (t, *J* = 8,1 Hz, 1H), 7,79 (br s, 1H), 8,38 (d, J = 8,1 Hz, 1H), 9,27 (br s, 1H). ¹³C RMN (DMSO-d₆): δ 14,5, 34,5, 59,7, 98,8, 116,2, 120,0, 123,5, 128,1, 135,2, 149,4, 149,8, 160,9.

**Préparation du 2-méthylamino-1-aminoindolizine-3-carboxylate d'éthyle (8).** Dans un tricol de 250 mL, équipé d'une agitation magnétique, d'un thermomètre et d'un réfrigérant sont chargés successivement 150 mL d'éthanol, 1,5mL d'eau et 5 g de poudre de zinc.
Le milieu est porté au reflux et on coule une solution 100mg de chlorure d'ammonium dans 1,5ml d'eau. On introduit ensuite par petites portions à la spatule 0,5g (2,02 mmole) de 2-méthylamino-1-nitroso-indolizine-3-carboxylate d'éthyle en 20 minutes. En fin d'addition, 2 ml d'acide acétique sont ajoutés et le reflux est maintenu pendant une heure.
Les sels de zinc sont éliminés par filtration sur Celite. Les jus sont acidifiés à l'aide d'une solution d'isopropanol-chlorhydrique puis concentrés sous vide.
Le solide pâteux ainsi obtenu est repris avec un minimum de méthanol et précipité avec de l'éther diisopropylique.
Le solide blanc beige formé est essoré sur fritté n°4 puis séché sous vide en présence de P₂O₅. Après séchage, on récupère une masse de 0,4g de produit attendu.
Les analyses par spectrométrie de masse et spectroscopie RMN sont conformes :
Les ions quasi-moléculaires (MH)+ , (MNa)+ de la molécule attendue, C₁₂H₁₅N₃O₂, sont principalement détectés.

### Exemple 3 : synthèse du 2-(méthylsulfonyl)-1-amino-3-indolizinecarboxylate d'éthyle (12) (composé D):

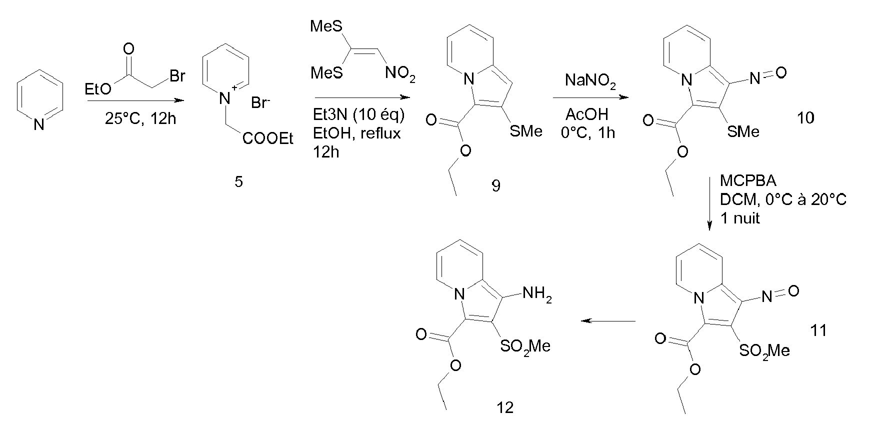

### Préparation du 2-(méthylsulfanyl)-3-indolizinecarboxylate d'éthyle (9). [1989, Journal of Heterocyclic Chemistry, p. 477]

Une solution de bromure de 1-éthoxycarbonylméthylpyridinium 5 (6,15 g, 25 mmoles), de 1,1-bis(méthylsulfanyl)-2-nitroéthylène (8,2 g, 50 mmoles) et 25 g de triéthylamine dans 150 ml d'éthanol est portée au reflux pendant 12 h. Après évaporation des solvants et de l'excès de triéthylamine, 150 ml d'eau sont ajoutés au résidu et la phase organique est extraite avec de l'éther de pétrole (75 ml x 3). Les extraits rassemblés sont séchés sur sulfate de magnésium anhydre, filtrés puis concentrés sous vide. L'huile résiduelle est chromatographiée sur colonne d'alumine neutre avec de l'hexane comme solvant d'élution pour conduire au 2-(méthylsulfanyl)-3-indolizinecarboxylate d'éthyle 9 (5,4g).

### Préparation du 2-(méthylsulfanyl)-1-nitroso-3-indolizinecarboxylate d'éthyle (10).

A une solution de 470 mg (2 mmoles) de 2-(methylsulfanyl)-3-indolizinecarboxylate d'éthyle 9 dans 10 ml d'acide acétique agitée à 0 °C, une solution de 165 mg de nitrite de sodium dans 5 ml d'eau est ajoutée goutte à goutte en 10 minutes. On laisse ensuite agiter le milieu réactionnel pendant une heure supplémentaire. La phase acide est basifiée avec une solution de NaOH 2N, puis extraite avec du dicholorométhane (25 ml x 3). La phase organique est séchée sur sulfate de magnésium, filtrée puis concentrée sous vide pour conduire au 2-(méthylsulfanyl)-1-nitroso-3-indolizinecarboxylate d'éthyle 10 (460 mg). PF : 134-135°C. ¹H RMN (CDCl₃): δ 9,58 (d, J = 6,8 Hz, 1H), 8,45 (d, J = 8,5 Hz, 1H), 7,70 (t, J = 8,2 Hz, 1H), 7,20 (td, J = 7,0, 1,1 Hz, 1H), 4,50 (q, J = 7,1 Hz, 2H), 3,05 (s, 3H), 1,52 (t, J = 7,1 Hz, 3H). ¹³C RMN (CDCl₃): 161,2, 156,9, 143,4, 135,7, 127,2, 123,7, 118,7, 118,0, 112,9, 61,2, 19,4, 14,4.

### Préparation du 2-(méthylsulfonyl)-1-nitroso-3-indolizinecarboxylate d'éthyle (11).

A une solution sous agitation de 2-(méthylsulfanyl)-1-nitroso-3-indolizinecarboxylate d'éthyle 10 (265 mg, 1 mmole) dans le dicholorométhane (5 mL) at 0 °C, de l'acide m-chloroperbenzoique (430 mg, à 65 %, 2 éq.) est ajouté et le mélange reactionnel est agité à 25 °C pendant une nuit. Il est ensuite lavé avec une solution saturée de bisulfite de sodium (3x 5 ml) puis une solution saturée de bicarbonate de soude (3x 5 ml). La phase organique est séchée sur sulfate de magnésium puis concentrée pour donner 290 mg de 2-(methylsulfonyl)-1-nitroso-3-indolizinecarboxylate d'éthyle **11** après recristallisation dans le méthanol. PF. 143-144 °C. ¹H RMN (CDCl₃): δ 9,16 (d, J = 7,0 Hz, 1H), 8,49 (d, J = 9,1 Hz, 1H), 7,60 (t, J = 8,0 Hz, 1H), 7,19 (t, J = 7,0 Hz, 1H), 4,58-4,47 (m, 2H), 3,27 (s, 3H), 1,49 (t, J = 7,1 Hz, 3H). ¹³C RMN (CDCl₃): δ 160,0, 136,8, 133,0, 129,7, 127,0, 118,8, 116,8, 115,0, 62,6, 39,7, 14,2.

La préparation du 2-(méthylsulfonyl)-1-amino-3-indolizinecarboxylate d'éthyle (12) à partir du 2-(méthylsulfonyl)-1-nitroso-3-indolizinecarboxylate d'éthyle (11) est effectuée selon un mode opératoire conforme à celui mis en oeuvre dans la dernière étape de l'exemple 1.

### Exemple 4 : synthèse de la 2-méthyl-1-aminoindolizine (14) (composé A):

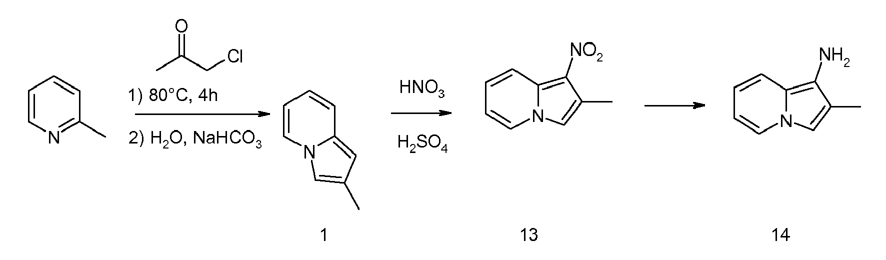

### Préparation de la 2-méthyl-1-nitroindolizine (13).

De l'acide nitrique (5 mL, 70%) est ajouté lentement à une solution sous agitation de 2-methylindolizine 1 (voir préparation de l'exemple 1) (5g, 38,2 mmoles) dans l'acide sulfurique concentré (15 mL, 96%) à 0° C. Après 5 minutes la solution brune obtenue est versée sur de la glace pilée. Le précipité orange formé est immédiatement filtré, lavé abondamment avec de l'eau, puis séché sous vide en présence P₂O₅ pendant une nuit pour obtenir la 2-méthyl-1-nitroindolizine **13** (3g), PF. 137-139 °C. ¹H RMN (DMSO-d₆): δ 8,55 (dt, J = 6,7, 1,0 Hz, 1H), 8,20 (d, J = 9,1 Hz, 1H), 7,54-7,48 (m, 2H), 7,11 (dt, J = 8,0, 1,2 Hz, 1H), 2,47 (d, J = 1,0 Hz, 3H). ¹³C RMN (DMSO-d₆): δ 132,2, 128,0, 127,7, 123,0, 122,8, 117,8, 115,0, 114,7, 12,4.

La préparation de la 2-méthyl-1-aminoindolizine (14) est effectuée par réduction de la 2-méthyl-1-nitroindolizine (13).

### Exemple 5 :synthèse de la 2,3-Dimethyl-indolizin-1-ylamine (17) (composé C)

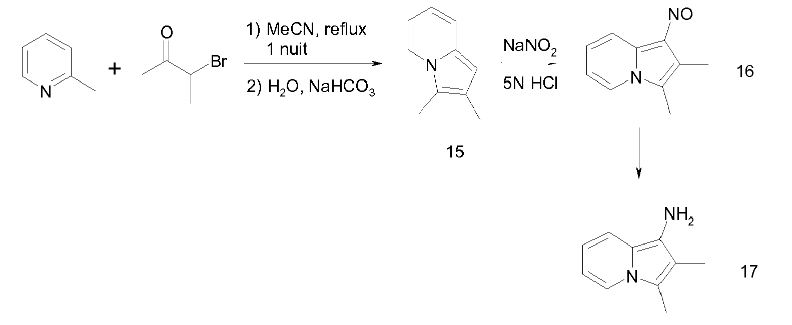

### Préparation de la 2,3-diméthylindolizine (15) [1962, Journal of the Chemical Society, p.2627].

Un mélange d'α-picoline (3g, 32,3 mmoles) et de 3-bromobutan-2-one (5g, 33 mmoles) est chauffé à 80°C pendant 8h. Le mélange réactionnel est versé sur un mélange chloroforme-eau (50:50mL), décanté puis séparé. La phase aqueuse est traitée par du bicarbonate de soude (14g, 166 mmoles) puis distillée pour conduire à un mélange de solide blanc et d'eau. L'extraction du mélange avec de l'éther diéthylique permet d'obtenir, après élimination des solvants, 4g de 2,3-dimethylindolizine 15 sous forme de cristaux blancs ; PF. 39-40 °C. ¹H RMN (CDCl₃): δ 7,60 (d, J = 6,9 Hz, 1H), 7,29-7,23 (m, 1H), 7,00-6,55 (m, 1H), 6,48-6,42 (m, 1H), 6,26 (s, 1H), 2,35 (s, 3H), 2,29 (s, 3H).

La préparation de la 2,3-Dimethyl-indolizin-1-ylamine (17) à partir de la 2,3-diméthylindolizine (15) s'effectue de la même façon que la préparation du composé (4) à partir du composé (2) de l'exemple 1.

### Exemples de formulations

Les exemples qui suivent présentent des compositions tinctoriales en milieu basique ou acide.

Pour les compositions tinctoriales en milieu basique, le support de teinture (1) de pH 9,5 a été utilisé :

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| NH₄Cl | 4,32 g |
| Ammoniaque à 20% de NH₃ | 2,94 g |

Pour les compositions tinctoriales en milieu acide, le support de teinture (2) de pH 7 a été utilisé :

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| Na₂HPO₄ | 0,28 g |
| KH₂PO₄ | 0,46 g |

Les compositions des exemples 1 à 8 comprennent du 2-méthyl-1-aminoindolizine-3- carboxylate de méthyle (composé B)

Au moment de l'emploi, chaque composition est mélangée avec un poids égal d'eau oxygénée à 20 volumes (6% en poids). On obtient un pH final de 9,5 ou 7 en fonction du support utilisé.

Chaque mélange obtenu est appliqué sur des mèches de cheveux naturels gris à 90 % blancs. Après 30 minutes de pose, les mèches sont rincées, lavées avec un shampooing standard, rincées à nouveau puis séchées.

### EXEMPLES 1 A 4 DE TEINTURE EN MILIEU BASIQUE pH 9,5:

| **Exemples** | **1** | **2** | **3** | **4** |
|---|---|---|---|---|
| Composé B | 10⁻³ mole | 10⁻³ mole | 10⁻³ mole | 10⁻³ mole |
| 5-Amino-2-methyl-phenol | 10⁻³ mole | | | |
| 1H-Indol-6-ol | | 10⁻³ mole | | |
| 2-(2,4-Diamino-phenoxy)-ethanol,chlorhydrate | | | 10⁻³ mole | |
| 3-Amino-2-chloro-6-methyl-phénol, chlorhydrate | | | | 10⁻³ mole |
| Support de teinture (1) | (*) | (*) | (*) | (*) |
| Eau déminéralisée q.s.p. | 100g | 100g | 100g | 100g |
| | | | | |

| **Exemple** | **1** | **2** | **3** | **4** |
|---|---|---|---|---|
| Nuance observée | gris violet | orangé | vert-bleu | gris bleu |

| | | | | |
|---|---|---|---|---|
| (*) Support de teinture (1) : voir plus haut | | | | |

### EXEMPLES 5 A 8 DE TEINTURE EN MILIEU ACIDE (pH = 7) :

| **Exemples** | **5** | **6** | **7** | **8** |
|---|---|---|---|---|
| Composé B | 10⁻³ mole | 10⁻³ mole | 10⁻³ mole | 10⁻³ mole |
| Benzene-1,3-diol | 10⁻³ mole | | | |
| 5-Amino-2-methyl-phenol | | 10⁻³ mole | | |
| 2-(2,4-Diamino-phenoxy)-ethanol,chlorhydrate | | | 10⁻³ mole | |
| 3-Amino-2-chloro-6-methyl-phénol, chlorhydrate | | | | 10⁻³ mole |
| Support de teinture (2) | (*) | (*) | (*) | (*) |
| Eau déminéralisée q. s. p. | 100g | 100g | 100g | 100g |
| | | | | |

| **Exemple** | **5** | **6** | **7** | **8** |
|---|---|---|---|---|
| **Nuance observée** | gris | gris | vert-bleu | gris bleu |

| | | | | |
|---|---|---|---|---|
| (*) Support de teinture (2) : voir plus haut | | | | |

Les compositions des exemples 9 à 16 comprennent du 2-méthyl-1-aminoindolizine (14) (composé A):

### EXEMPLE 9 DE TEINTURE EN MILIEU BASIQUE (pH 9,5):

| **Exemple** | **9** |
|---|---|
| Composé A | 10⁻³ mole |
| 3-Amino-2-chloro-6-methyl-phenol,chlorhydrate | 10⁻³ mole |
| Support de teinture (1) | (*) |
| Eau déminéralisée q.s.p. | 100g |
| | |

| **Exemple** | **9** |
|---|---|
| **Nuance observée** | gris jaune-vert |

| | |
|---|---|
| (*) Support de teinture (1) : voir plus haut | |

| **Exemples** | **10** | **11** | **12** | **13** | **14** |
|---|---|---|---|---|---|
| Composé A | 10⁻³ mole | 10⁻³ mole | 10⁻³ mole | 10⁻³ mole | 10⁻³ mole |
| Benzene-1,3-diol | 10⁻³ mole | | | | |
| 5-Amino-2-methyl-phenol | | 10⁻³ mole | | | |
| 1H-Indol-6-ol | | | mole | | |
| 2-Amino-pyridin-3-ol | | | | 10⁻³ mole | |
| 3,6-Dimethyl-1H-pyrazolo[5,1-c][1,2 ,4]triazole | | | | | 10⁻³ mole |
| Support de teinture (2) | (*) | (*) | (*) | (*) | (*) |
| Eau déminéralisée q.s.p. | 100g | 100g | 100g | 100g | 100g |
| | | | | | |

| **Exemples** | **10** | **11** | **12** | **13** | **14** |
|---|---|---|---|---|---|
| **Nuance observée** | jaune | brun | orangé | orangé | Orangé |

| | | | | | |
|---|---|---|---|---|---|
| (*) Support de teinture (2) : voir plus haut | | | | | |

| **Exemples** | 15 | 16 |
|---|---|---|
| Composé A | 10⁻³ mole | 10⁻³ mole |
| 2-(2,4-Diamino-phenoxy)-ethanol,chlorhydrate | 10⁻³ mole | |
| 3-Amino-2-chloro-6-methyl-phenol,chlorhydrate | | 10⁻³ mole |
| Support de teinture (2) | (*) | (*) |
| Eau déminéralisée q.s.p. | 100g | 100g |
| | | |

| **Exemples** | 15 | 16 |
|---|---|---|
| **Nuance observée** | gris | gris |

| | | |
|---|---|---|
| (*) Support de teinture (2) : voir plus haut | | |

## Revendications

1. Aminoindolizine de formule générale (I), ses sels d'addition avec un acide et ses solvates : 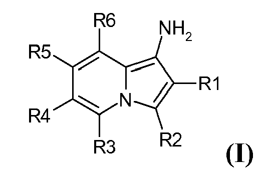 dans laquelle
• R₁ est un radical choisi parmi :
- les radicaux alkyles en C₁-C₁₀, linéaires ou ramifiés, éventuellement substitués par un ou plusieurs groupements OH ou OR₁₉, R₁₉ étant choisi parmi les radicaux alkyles en C₁-C₆, linéaires ou ramifiés, éventuellement substitués par un ou plusieurs radicaux OH,
- les radicaux soufrés choisis parmi SR, SO₂Me, R étant un radical alkyle en C₁-C₆, linéaire ou ramifié, ou un radical aryle,
- les radicaux azotés choisis parmi NH₂, NHR₇ ou NR₇R₈, R₇ et R₈ identiques ou différents, étant choisis parmi les radicaux alkyles en C₁-C₆, linéaires ou ramifiés, éventuellement substitués par un ou plusieurs groupes hydroxy, amino, mono- ou dialkylamino en C₁-C₆, carboxamido, ureido ou guanidinyle, éventuellement interrompus par un atome d'oxygène ou un ou plusieurs groupes N(R₁₃), R₁₃ étant un atome d'hydrogène ou un radical alkyle en C₁-C₆, les radicaux R₇ et R₈ pouvant former ensemble avec l'atome d'azote auquel ils sont rattachés un hétérocycle insaturé ou saturé comprenant de 5 à 7 chaînons,
- le radical OR₉, R₉ étant choisi parmi les radicaux alkyles en C₁-C₆, linéaires ou ramifiés, éventuellement substitués par un ou plusieurs radicaux OH, ou les radicaux alcoxy en C₁-C₆, linéaires ou ramifiés,
- les radicaux choisis parmi COOH, CONH₂, CONHR₁₁, ou CONR₁₁R₁₂, R₁₁ et R₁₂, identiques ou différents, étant choisis parmi les radicaux alkyles en C₁-C₆, linéaires ou ramifiés, éventuellement interrompus par un ou plusieurs groupements N(R₁₄), R₁₄ étant un atome d'hydrogène ou un radical alkyle en C₁-C₆, éventuellement substitué par un groupement hydroxy, les radicaux R₁₁ et R₁₂ pouvant former ensemble et avec l'atome d'azote auquel ils sont rattachés un hétérocycle insaturé ou saturé comprenant de 5 à 7 chaînons,
• R₂ est un radical choisi parmi :
- les radicaux alkyles en C₁-C₆, linéaires ou ramifiés,
- l'atome d'hydrogène,
- les radicaux choisis parmi COOH, CN, CF₃ ou alcoxycarbonyle COOR₁₀, R₁₀ étant choisi parmi les radicaux alkyles en C₁-C₆, linéaires ou ramifiés, les radicaux aryle ou hétéroaryle,
- les radicaux choisis parmi CONH₂, CONHR₁₁, ou CONR₁₁R₁₂,
- le radical OR₉.
- les radicaux SR, SOR, ou SO₂R,
R, R₉, R₁₁ et R₁₂ étant tels que définis précédemment,
• R₃, R₄, R₅, R₆, indépendamment l'un de l'autre, sont choisis parmi:
- les radicaux aryles, hétéroaryles, ou alkyles en C₁-C₆, linéaires ou ramifiés, éventuellement interrompus par un ou plusieurs groupements N(R₁₃) ou par un oxygène,
- le radical OR₉,
- l'atome d'hydrogène,
- les halogènes choisis parmi le fluor, le chlore, ou le brome.
- les radicaux azotés choisis parmi NH₂, NHR₇ ou NR₇R₈,
- les radicaux COOH, CN ou alcoxy carbonyle COOR₁₀,
R₇, R₈, R₉, R₁₀, étant tels que définis précédemment,
à la condition qu'au plus un seul des radicaux R₁, R₃, R₄, R₅, R₆ représente un radical azoté choisis parmi NH₂, NHR₇ ou NR₇R₈.

2. Aminoindolizine de formule générale (I) selon la revendication 1 telle que le radical R₁ est choisi parmi :
- les radicaux alkyles en C₁-C₁₀, linéaires ou ramifiés, de préférence C₁-C₆ et plus préférentiellement C₁-C₄,
- le radical soufré SO₂Me,
- les radicaux azotés choisis parmi NHR₇ ou NR₇R₈, R₇ et R₈ identiques ou différents, étant choisis parmi les radicaux alkyles en C₁-C₆, linéaires ou ramifiés, éventuellement substitués par un ou plusieurs groupes hydroxy, amino, mono- ou dialkylamino en C₁-C₆, de préférence hydroxy, mono ou dialkylamino en C₁-C₄.

3. Aminoindolizine de formule générale (I) selon la revendication 2 telle que le radical R₁ est choisi parmi les radicaux suivants : -CH₃, -SO₂CH₃, -NH(CH₃), -N(CH₂CH₂OH)₂, -N(CH₃)₂, - NH(CH₂CH₂OH), -NH(CH₂CH₂N(CH₃)₂), -NH(CH₂CH₃), - NH(CH₂CH₂CH₂N(CH₃)₂).

4. Aminoindolizine de formule générale (I) selon l'une quelconque des revendications précédentes telle que le radical R₂ est choisi parmi :
- les radicaux alkyles en C₁-C₆, linéaires ou ramifiés, de préférence C₁-C₄,
- l'atome d'hydrogène,
- les radicaux choisis parmi COOH, CN, CF₃ ou alcoxycarbonyle COOR₁₀, R₁₀ étant choisi parmi les radicaux alkyles en C₁-C₆, linéaires ou ramifiés,
- les radicaux choisis parmi CONH₂, CONHR₁₁, ou CONR₁₁R₁₂,
- le radical OR₉,
- les radicaux SR ou SO₂R,
R₉, R₁₀, R₁₁ et R étant définis à la revendication 1.

5. Aminoindolizine de formule générale (I) selon la revendication précédente telle que le radical R₂ est choisi parmi l'atome d'hydrogène ou les radicaux suivants: -CH₃, -COOCH₃, -COOCH₂CH₃, - CN, -CF₃, - CONH(CH₂CH₃), -CONH(CH₂CH₂CH₂N(CH₃)₂), - CON(CH₃)₂, -CONH(CH₂CH₂OH), -CONH₂, -OCH₃, -SCH₃, -SO₂CH₃.

6. Aminoindolizine de formule générale (I) selon l'une quelconque des revendications précédentes telle que les radicaux R₃, R₄, R₅, R₆, indépendamment l'un de l'autre, sont choisis parmi:
- l'atome d'hydrogène,
- le radical OR₉ avec R₉ étant un radical alkyle C₁-C₄, de préférence - OCH₃,
- le radical -NH₂, à la condition qu'au plus un seul des radicaux R₁, R₃, R₄, R₅, R₆ représente un radical -NH₂.

7. Aminoindolizine de formule générale (I) selon l'une quelconque des revendications précédentes telle qu'elle est choisie parmi : 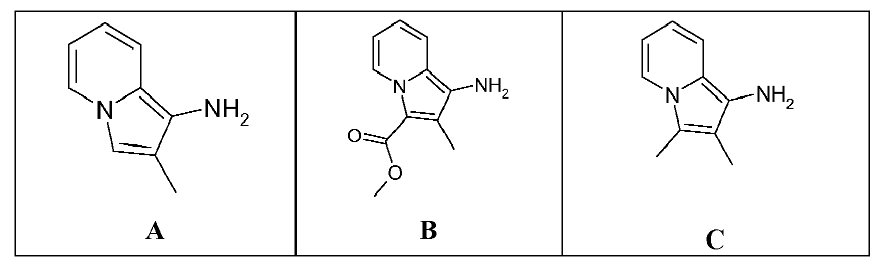 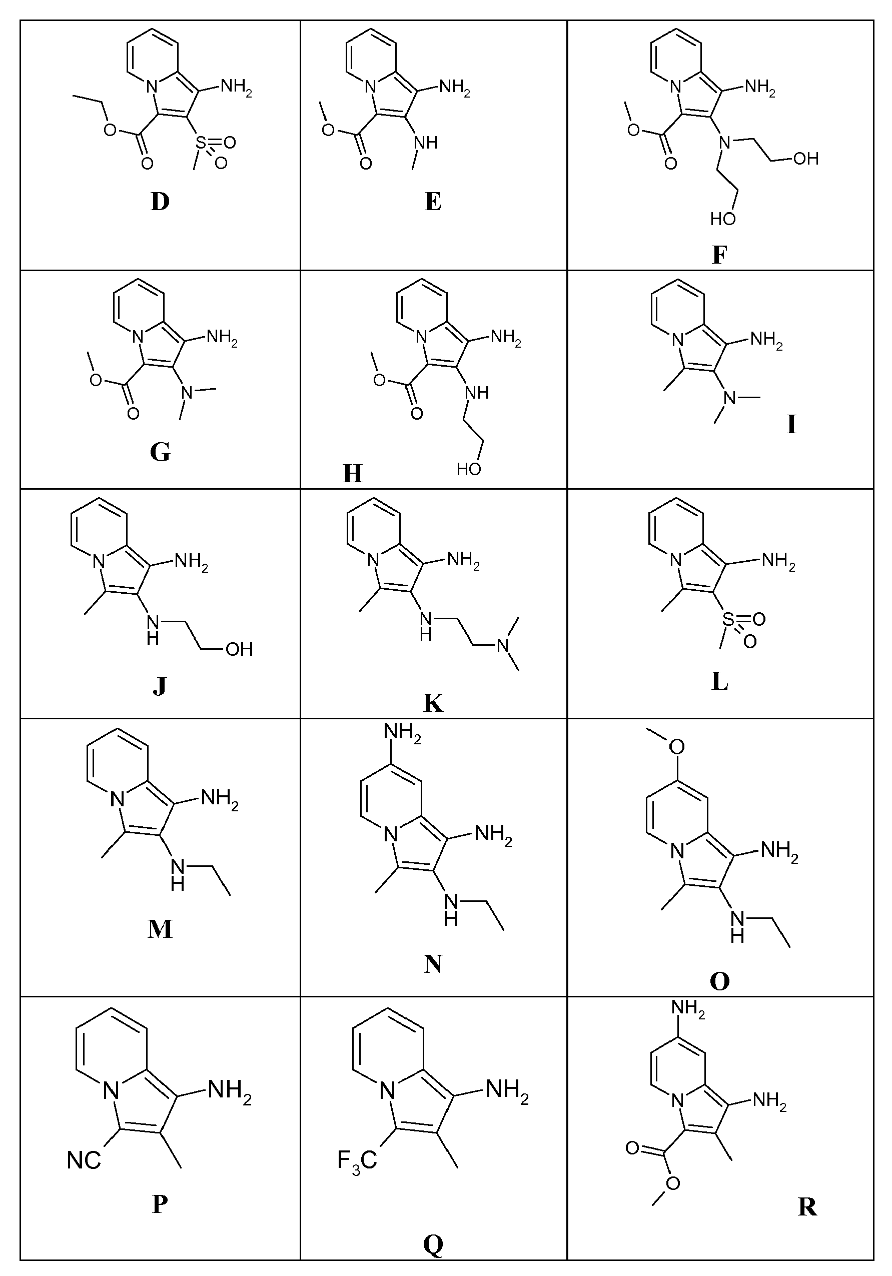 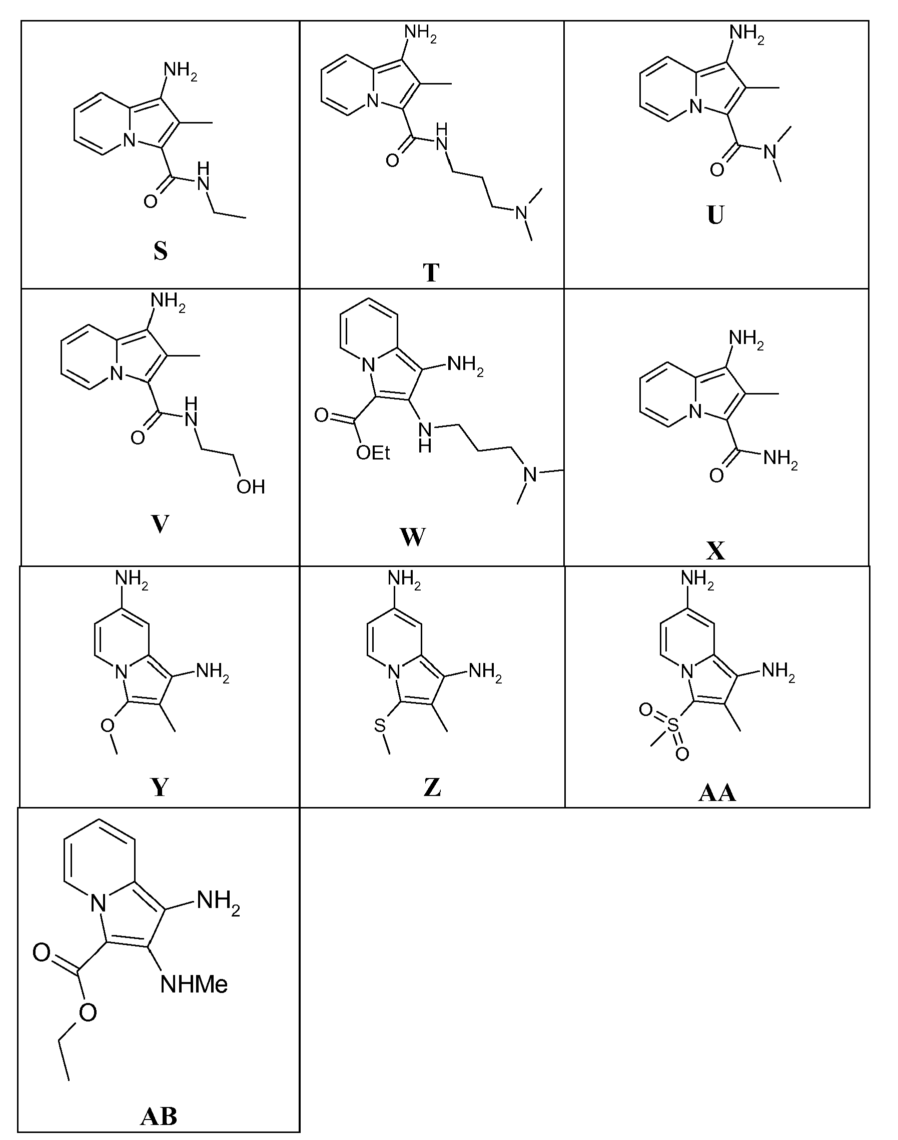 ainsi que leurs sels d'addition et/ou solvates.

8. Utilisation d'une aminoindolizine de formule générale (I) et/ou ses sels ou solvates telle que définie à l'une quelconque des revendications 1 à 7 pour la teinture des fibres kératiniques, notamment les fibres kératiniques humaines telles que les cheveux.

9. Composition cosmétique de teinture comprenant dans un milieu approprié pour la teinture, au moins une aminoindolizine de formule générale (I) et/ou ses sels ou solvates telle que définie à l'une quelconque des revendications 1 à 7.

10. Composition selon la revendication précédente **caractérisée par le fait que** la concentration en aminoindolizine de formule générale (I) est comprise entre 0,0001 et 20%, de préférence entre 0,005 à 6% en poids par rapport au poids total de la composition

11. Composition selon la revendication 9 ou 10 **caractérisée par le fait qu'**elle comprend au moins un composé choisi parmi les bases d'oxydation additionnelles différentes des aminoindolizines de formule (I), les coupleurs d'oxydation et les colorants directs naturels ou cationiques.

12. Composition selon l'une quelconque des revendications 9 à 11 **caractérisée en ce qu'**il s'agit d'une composition prête à l'emploi comprenant au moins un agent oxydant choisi parmi le peroxyde d'hydrogène, le peroxyde d'urée, les bromates de métaux alcalins, les persels, les peracides et les enzymes oxydases.

13. Procédé de teinture des fibres kératiniques **caractérisée par le fait qu'**on applique sur lesdites fibres la composition selon l'une des revendications 9 à 11, pendant une durée suffisante pour développer la coloration désirée en présence d'un agent oxydant, l'agent oxydant étant appliqué avant, simultanément ou après la composition.

14. Procédé de teinture des fibres kératiniques **caractérisée par le fait qu'**on applique sur lesdites fibres la composition prête à l'emploi selon la revendication 12, pendant une durée suffisante pour développer la coloration désirée.

15. Dispositif à plusieurs compartiments, un premier compartiment contenant la composition cosmétique pour la teinture des fibres kératiniques telle que définie à l'une quelconque des revendications 9 à 11 et un deuxième compartiment contenant un agent oxydant.
